# EUROPÄISCHE PATENTSCHRIFT

(11) **EP 1 139 791 B1**
(45) Veröffentlichungstag und Bekanntmachung des Hinweises auf die Patenterteilung: **13.10.2004**
(21) Anmeldenummer: 99965441.1
(22) Anmeldetag: 08.12.1999
(51) Int. Cl.: A23L 1/22, B01J 13/04, B01J 2/16

(54) **EINGEKAPSELTE AROMA- UND/ODER RIECHSTOFFZUBEREITUNGEN**
ENCAPSULATED FLAVOUR AND/OR FRAGRANCE PREPARATIONS
PREPARATIONS D'AROMES ET/OU DE PARFUMS ENCAPSULEES

(30) Priorität: 18.12.1998 DE 19858729; 25.11.1999 DE 19956604
(43) Veröffentlichungstag der Anmeldung: 10.10.2001
(73) Patentinhaber: Symrise GmbH & Co. KG, 37603 Holzminden (DE)
(72) Erfinder: SCHLEIFENBAUM, Birgit, D-37671 Höxter (DE); UHLEMANN, Jens, D-37603 Holzminden (DE); BOECK, Reinhard, D-41564 Kaarst (DE); HINDERER, Jürgen, D-51381 Leverkusen (DE)
(74) Vertreter: Eisenführ, Speiser & Partner
(86) Internationale Anmeldenummer: PCT/EP1999/009644
(87) Internationale Veröffentlichungsnummer: WO 2000/036931

(56) Entgegenhaltungen:
- WO-A-97/16078
- WO-A-98/00694
- UHLEMANN H: "KONTINUIERLICHE WIRBELSCHICHT-SPRUEHGRANULATION" CHEMIE. INGENIEUR. TECHNIK,DE,VERLAG CHEMIE GMBH. WEINHEIM, Bd. 62, Nr. 10, 1990, Seiten 822-834, XP000176381 ISSN: 0009-286X in der Anmeldung erwähnt

## Beschreibung

Die vorliegende Erfindung betrifft mittels kontinuierlicher Wirbelschichtsprühgranulation eingekapselte Aroma- und/oder Riechstoffzubereitungen, ein Verfahren zu ihrer Herstellung und ihre Verwendung vorzugsweise in Lebensmitteln.

Bei Aromen (Geschmacksstoffen) und Riechstoffen handelt es sich um komplexe flüssige Mischungen in der Regel flüchtiger Komponenten. Aromagranulate werden für verschiedene Zwecke benötigt. Üblich ist die Aromeneinkapselung über Sprühtrocknung, bei der jedoch nur verhältnismäßig feine und unregelmäßig strukturierte Partikel erzeugt werden (R. Büttiker, Dissertation ETH Zürich Nr. 6148).

Eine Alternative zur Sprühtrocknung ist die Herstellung von Aromagranulaten über Wirbelschichtsprühgranulation. Die EP A 070 719 beschreibt z.B. die Herstellung von Aromagranulaten in einer konventionellen diskontinuierlich betriebenen Wirbelschicht. Dabei wird eine Emulsion der zu granulierenden Aromen in ein Wirbelbett gesprüht. welches aus mit Luft aufgewirbelten Partikeln besteht. Die Partikel wirken dann als Keime für die Bildung der Granulatkörner.

In der WO 97/16078 wird die Herstellung von Aromagranulaten in einem konventionellen diskontinuierlich betriebenen Fließbettrotorgranulator beschrieben. Durch eine rotierende Bodenplatte erzeugt der Rotorgranulator eine Verwirbelung des in ihm enthaltenen Fließbettes. Bei diesem Verfahren kann nur auf vorgelegte Kerneaufgesprüht werden, so dass der Aromagehalt des Endproduktes sehr niedrig ist.

Beide Verfahren arbeiten diskontinuierlich, d.h. nach der Verarbeitung eines Sprühlösungsansatzes ist der Herstellungsprozeß beendet und das Granulat wird der Vorrichtung entnommen. Anschließend muß ein neuer Sprühlösungsansatz verarbeitet werden. Aus Gründen der Wirtschaftlichkeit müssen die Verfahren daher mit einem hohen Bettinhalt betrieben werden. Für ein vorgegebenes Granulatwachstum muß eine entsprechende Menge von Sprühlösung verdampft werden. Deshalb führt der hohe Bettinhalt zu langen Verweilzeiten des Granulates, die im Bereich von Stunden liegen. Eine lange Verweilzeit führt jedoch bei gleichzeitiger Temperaturbeanspruchung der Granulate in einem Luftstrom zu entsprechend hohen Verlusten bei den flüchtigen Aromen. Eine Absenkung von Lufttemperatur und/oder des Luftdurchsatzes verringert den Aromenverlust nicht, da sich dann zwangsläufig die Zeit zur Verdampfung der Sprühlösung verlängert.

Ein weiterer Nachteil des Verfahrens nach der EP A 070 719 ist, dass die damit erhaltenen Produkte zur Herstellung von engen Korngrößenverteilungen nachgesiebt werden müssen. Das ist zum einen ein zusätzlicher Arbeitsaufwand, zum anderen geht dabei wertvolles Material verloren.

Nachteilig bei WO 97/16078 ist der große Anteil von Füllmaterial im Granulatkern (ca. 60 bis 90 Gew.-%) und die nur oberflächliche Anlagerung der Aromen an die Granulate. Die oberflächliche Anlagerung vermindert den Schutz der Aromen, limitiert die maximale Beladung und führt zu unerwünscht hohen Anteilen von Aromen an der Oberfläche des Granulats.

Zwar können die gemäß EP A 070 719 oder WO 97/16078 hergestellten Granulate mit einer Umhüllung versehen werden, um das Löslichkeits- und Aromafreisetzungsverhalten einzustellen, oder um eine gezielte Schutzwirkung zu erreichen. Die noch immer verhältnismäßig uneinheitliche Korngrößenverteilung und die unregelmäßige Granulatoberfläche erschweren jedoch eine gleichmäßige Umhüllung mit konstanter Hülldicke. Eine wirklich zeit- oder temperaturkontrollierte Freisetzung der Aromen bzw. der Riechstoffe kann so nicht erreicht werden.

Ziel der vorliegenden Erfindung sind granulierte eingekapselte Aroma- und/oder Riechstoffzubereitungen. Die Granulate sollten eine einstellbare Korngröße, vorzugsweise im Bereich von 0,2 bis 2 mm, mit enger Korngrößenverteilung und kugeliger Geometrie, sowie eine hohe Beladung mit flüchtigen Aromen aufweisen. Bei der Herstellung soll die Retention der flüchtigen Aromen maximiert, die Siebverluste minimiert und die Ausbeute für eine einstellbare Wunschkomgröße maximiert werden können. Die Granulate sollten weiterhin ideale Voraussetzungen für eine Umhüllung aufweisen, damit durch geschickte Wahl der Umhüllung die Aromaeigenschaften gezielt auf einen Verwendungszweck abgestimmt werden können.

Es wurden eingekapselte Aroma- und/oder Riechstoffzubereitungen gefunden, hergestellt mittels kontinuierlicher Wirbelschichtsprühgranulation, bei der eine Aroma- und/oder Riechstoffzubereitung in ein Wirbelbett mit darin erzeugten Granulationskeimen eingesprüht wird und bei der die durchschnittliche Verweilzeit der eingesprühten Aromen- und/oder Riechstoffzubereitung im Wirbelbett weniger als 20 Minuten beträgt.

Die erfindungsgemäßen neuen Aroma- und/oder Riechstoffzubereitungen erfüllen die oben genannten Forderungen. Insbesondere weisen sie Korngrößen von 0,2 bis 2 mm auf, sie sind staubfrei, die Aromenbeladungen liegen im Bereich von 1 bis 25 Gew.-%, die Retentionen der Aromen während des Granulationsprozesses liegen im Bereich von 60 bis 90 Gew.-%.

Es wurde auch ein Verfahren zur Herstellung von eingekapselten Aroma- und/oder Riechstoffzubereitungen, hergestellt mittels Wirbelschichtsprühgranulation, bei der eine Aromen- und/oder Riechstoffzubereitung in ein Wirbelbett mit darin erzeugten Granulationskeimen eingesprüht wird, gefunden, dadurch gekennzeichnet, dass die durchschnittliche Verweilzeit der eingesprühten Aromen- und/oder Riechstoffzubereitung im Wirbelbett weniger als 20 Minuten beträgt.

Die durchschnittliche Verweilzeit der eingesprühten Aromen- und/oder Riechstoffzubereitung im Wirbelbett beträgt bevorzugt 2 bis 15 Minuten, insbesondere 5 bis 10 Minuten.

In Untersuchungen hat sich gezeigt, dass sich einerseits Aromen- und/oder Riechstoffe in einer derart kurzen Zeitspanne ausreichend granulieren lassen, und dass dabei andererseits ein Produkt erhalten wird, das hinsichtlich der Verteilung der Korngrößen, der Geometrie, der Retention sowie Beladung erheblich verbessert ist. Eine hohe Beladung bedeutet dabei eine große Gesamtmenge von eingekapseltem Aroma bezogen auf die Granulatmasse. Je höher die Retention der einzelnen flüchtigen Komponenten ist, je niedriger sind die Verluste dieser Komponente.

Das erfindungsgemäße Verfahren kann diskontinuierlich und kontinuierlich ausgeführt werden. Vorzugsweise wird das erfindungsgemäße Verfahren kontinuierlich ausgeführt. Ein kontinuierliches Verfahren ist besser für eine industrielle Produktion geeignet, und weist kurze Verweilzeiten auf. Bei gleichem Materialdurchsatz ist der Bettinhalt bei den kontinuierlichen Verfahren der Wirbelschichtsprühgranulation niedriger als bei den diskontinuierlichen Verfahren. Statt die Gesamtmenge aller Granulatkeime gleichzeitig aufwachsen zu lassen, wird bei der kontinuierlichen Wirbelschichtsprühgranulation nur eine kleine Menge der Granulatkeime besprüht und nach Erreichen der gewünschten Korngröße sofort über einen Sichter ausgetragen. Die erfindungsgemäß hergestellten eingekapselten Aroma und/oder Riechstoffzubereitungen weisen eine geringe Korngrößenverteilung auf; außerdem können gezielt die Körner der passenden Größe entnommen werden.

Im Rahmen der vorliegenden Erfindung ist es daher bevorzugt. dass das Wirbelbett eine geringe Betthöhe hat. Vorzugsweise beträgt diese 3 bis 50 cm, insbesondere bevorzugt 5 bis 20 cm.

Durch kontinuierliche Wirbelschichtsprühgranulation entstehen aus einer Sprühlösung bestehend aus Wasser, emulgiertem Aroma und gelösten/suspendierten Trägerstoffen freifließende, staubarme, körnige Granulate mit eingekapseltem Aroma/Riechstoff umgewandelt. Dabei erfolgen im Idealfall die Grundvorgänge Keimerzeugung, Trocknen, Formgebung und selektives Austragen der Granulate, welche die Wunschkorngröße erreicht haben, simultan in einem Apparat.

Das Grundprinzip der kontinuierlichen Wirbelschichtsprühgranulation (Chemie-Ingenieur-Technik, 62. Jahrgang (1990), Seiten 822 bis 834) ist in zahlreichen Varianten realisiert worden. Zu unterscheiden sind insbesondere die Variante mit externer Keimbildung, bei denen Keime aus externen Sichtern, Mahlanlagen oder sonstigen Feststoffspeichern in das Bett dosiert werden, sowie Varianten mit interner Keimbildung. Im Vergleich weisen die Varianten mit externer Keimbildung allerdings aus zwei Gründen eine erhöhte Verweilzeit auf:
1. Die Betthöhe wird über die Keimzufuhr geregelt und kann deshalb nicht unter ein regelbares Minimum abgesenkt werden.
2. Es werden für das Verfahren externe Feststoffkreisläufe benötigt.

Im Rahmen der vorliegenden Erfindung werden daher Verfahren mit interner Keimzufuhr beansprucht. Ein solches ist z.B. das in der EP A 163 836 beschriebene Verfahren. Dieses verfügt weiterhin über einen selbstregelnden Mechanismus zur Korngrößenregelung und weist daher eine minimale Verweilzeit auf.

Die Sprühlösung kann von unten, von der Seite, aber auch von oben in die Wirbelschicht gesprüht werden. Für die Abtrennung von mitgerissenen Feststoffen aus der Abluft sind zahlreiche Varianten möglich, die sich durch das Abscheideverfahren (z.B. Zyklon oder Filter) oder durch den Ort der Abtrennung (innerhalb oder außerhalb des Granulators) unterscheiden.

Schließlich werden für das Austragen der Granulate vorzugsweise Sichter verwendet. Mit den Sichtern wird erreicht, dass nur die groben Partikel die Wirbelschicht verlassen können. Die übrigen Partikel bleiben solange in der Wirbelschicht zurück, bis auch sie die Wunschkorngröße erreicht haben.

Die Partikel des Granulates werden vorzugsweise nach der Erzeugung mit einer Umhüllung versehen. Diese Umhüllung kann entweder in dazu geeigneten Wirbelschichtapparaturen (Top-Spray-Coater, Bottom-Spray-Coater, Wurster-Coater) oder in Filmcoatern aufgetragen werden. Dies geschieht durch Aufsprühen einer Lösung, Emulsion, Dispersion oder Schmelze einer Substanz, die für diese Zwecke aufgrund ihrer filmbildenden Eigenschaften bekanntermaßen verwendet wird. Als Umhüllungsmaterialien können Substanzen bzw. Substanzgemische wie z.B. Fette, Wachse, Proteine, wie Gelatine, Hydrokolloide wie Stärken, abgebaute Stärken, chemisch modifizierte Stärken, modifizierte Cellulosen, mikrokristalline Cellulose, pflanzliche exudate, wie Gummi Arabicum, Ghatti-Gummi, Traganth, Karaya, Pflanzenextrakte, wie Carrageenan, Guarkemmehl, Johannisbrotkernmehl, Agar-Agar, Alginate, Pektin, Inulin, Tierextrakte wie Chitosan und Schellack, Produkte aus Mikroorganismen, wie Xanthan Gum, Gellan Gum, kosmetisch oder pharmazeutisch anwendbare Kunstoffe, z.B. Polyvinylpyrrolidon, Polyacrylate, Polymethacrylate, Polyvinylacetatphthalat, Polyethylenglykol verwendet werden. Das Umhüllungsmaterial wird auf die jeweiligen Anforderungen an das Granulat je nach Verwendungszweck abgestimmt.

Die zu granulierende Sprühlösung kann - analog zum Vorgehen bei der Sprühtrocknung von Aromen - aus Wasser mit gelösten und/oder suspendierten polymeren Trägerstoffen und emulgiertem Aroma bestehen. Die polymeren Trägerstoffe können hydrolisierte oder modifizierte Stärken oder Hydrokolloide wie z.B. Gummi Arabikum als eine Stoffe oder in beliebigen Mischungsverhältnissen sein.

Der zu granulierenden Sprühlösung können auch übliche Zusatzstoffe und Zutaten wie Lebensmittel- oder Kosmetik-Farbstoffe, Süßstoffe, Antioxidantien. Genußsäuren wie Zitronensäure, geschmacksbeeinflussende Stoffe wie Natriumglutamat. Vitamine, Mineralstoffe, Saftkonzentrate etc. zugegeben werden.

Das erfindungsgemäße Verfahren der Wirbelschichtsprühgranulation wird vorzugsweise bei erhöhten Lufttemperaturen im Bereich von 60°C und 180°C, bevorzugt von 100°C und 140°C, durchgeführt. Der Luftdurchsatz wird für maximale Trocknungsleistung so groß wie möglich gewählt. Die geeigneten Gasgeschwindigkeiten liegen im Bereich von 0,5 bis 1,5 m/s, bevorzugt bei 1 m/s. Die zulässige Produkttemperatur ist gekoppelt an die Ablufttemperatur und wird über die Sprührate der Sprühlösung eingestellt. Die Betthöhe beträgt bei der Verfahrensweise gemäß EP A 163 836 nur wenige Zentimeter. Die Betthöhe bei den Varianten mit externer Keimbildung wird im Bereich von 20 bis 50 Zentimeter geregelt.

Als Aromen und Riechstoffe kommen komplexe Aroma- und Riechstoffkompositionen, die alle bisher für Aromen und Parfüms verwendete Einzelkomponenten, also Aroma und/oder Riechstoffe sowie etherische Öle oder Fraktionen daraus enthalten können, aber auch nur einzelne Aroma- oder Riechstoffe, z.B. Acetaldehyd, Menthol, Ethylbutyrat, etc., oder etherische Öle oder Fraktionen daraus in Frage.

Als Aromen- und Riechstoffe im Rahmen der vorliegenden Erfindung, seien bevorzugt beispielsweise genannt: Beeren, Citrus, Kernobst, Käse, Fleisch, Fisch, Meeresfrüchte, Gewürze, Kräuter, Gemüse, Kaffee, Schokolade, Mint. Tabak, Holz, Blumen, etc.

Die erfindungsgemäßen eingekapselten Aroma- und/oder Riechstoffzubereitungen können vorzugsweise in Lebensmitteln verwendet werden.

Im folgenden wird die Erfindung anhand von Beispielen erläutert.

### Herstellungsbeispiele

### Beispiel 1 (Erdbeere):

In einer Granulierapparatur des in EP 163 836 dargestellten Typs (mit den folgenden Merkmalen: Durchmesser Anströmboden: 225 mm, Sprühdüse: Zweistoffdüse; Sichtender Austrag: Zick-Zack-Sichter; Filter: internes Schlauchfilter) wird eine Lösung bestehend aus 44 Gew.-% Wasser, 11 Gew.-% Erdbeeraroma, 13 Gew.-% Gummi arabicum und 32 Gew.-% hydrolysierter Stärke (Maltodextrin DE 15-19) granuliert. Die Lösung wird bei einer Temperatur von 32°C in den Wirbelschichtgranulator gesprüht. Zur Fluidisierung des Bettinhaltes wird Stickstoff in einer Menge von 140 kg/h eingeblasen. Die Eintrittstemperatur des Fluidisiergases beträgt 140°C. Die Temperatur des Abgases beträgt 76°C. Als Sichtgas wird ebenfalls Stickstoff in einer Menge von 15 kg/h mit einer Temperatur von 50°C zugeführt. Der Inhalt des Wirbelbettes beträgt ca. 1700 g. Die Granulierleistung beträgt ca. 2.8 kg pro Stunde. Man erhält ein frei fließendes Granulat mit einem mittleren Teilchendurchmesser von 1 mm bei einer Schüttdichte von 600 g/l. Die Granulate sind rund und weisen eine glatte Oberfläche auf. Aufgrund des konstanten Druckverlustes des Filters und des ebenfalls konstant bleibenden Bettinhalts ist von stationären Bedingungen hinsichtlich des Granulationsprozesses auszugehen.

### Beispiel 2 (Mint):

In der in Beispiel "Erdbeere" beschriebenen Apparatur wird eine Granulierung einer Lösung bestehend aus 37 Gew.-% Wasser, 15 Gew.-% Gummi arabicum, 35 Gew.% hydrolysierter Stärke (Maltodextrin DE 15-19) und 13 Gew.-% Pfefferminzaroma durchgeführt. Die Lösung wird mit blauer Farbe (E131) ungefärbt (40 g einer 2%igen Lösung). Die Lösung wird bei einer Temperatur von 35°C in den Wirbelschichtgranulator gesprüht. Zur Fluidisierung des Bettinhaltes wird Stickstoff in einer Menge von 130 kg/h eingeblasen. Die Eintrittstemperatur des Fluidisiergases beträgt 140°C. Die Temperatur des Abgases beträgt 85°C. Als Sichtgas wird ebenfalls Stickstoff in einer Menge von 16 kg/h mit einer Temperatur von 30°C zugeführt. Der Inhalt des Wirbelbettes beträgt ca. 1700 g. Die Granulierleistung beträgt ca. 4 kg pro Stunde. Man erhält ein frei fließendes Granulat mit einem mittleren Teilchendurchmesser von 1 mm bei einer Schüttdichte von 550 g/l. Die Granulate sind rund und weisen eine rauhe Oberfläche auf.

In derselben Apparatur wurden die zuvor erzeugten Granulate mit dem Fett Revel A (von Fa. Loders Croklaan) gecoatet; als Bettvorlage werden 400 g vorgelegt. Durch Anheben der Sichtgasmenge auf 23 kg/h bei 25°C wird kein Material ausgetragen, d.h. das Coaten findet im Batchbetrieb statt. Die Schmelze wird bei einer Temperatur von 74°C in den Wirbelschichtgranulator gesprüht. Die Temperatur des Zerstäubungsgases beträgt 70°C. Zur Fluidisierung des Bettinhaltes wird Stickstoff in einer Menge von 100 kg/h eingeblasen. Die Eintrittstemperatur des gekühlten Fluidisiergases beträgt 16°C. Die Temperatur des Abgases beträgt 28°C. Man erhält ein frei fließendes Granulat. Die Granulate sind rund. REM-Aufnahmen der Bruchflächen zeigen eine weitgehend gleichmäßige Beschichtung der Granulate mit dem Fett.

### Beispiel 3 (Tee-Extrakt):

In der in Beispiel "Erdbeere" beschriebenen Apparatur wird eine Granulierung einer Lösung bestehend aus 25 Gew.-% Wasser, 4 Gew.-% Gummi arabicum, 19 Gew.-% hydrolysierter Stärke (Maltodextrin DE 15-19) und 52 Gew.-% Tee-Extrakt (Feststoffgehalt ca. 63 Gew.-%) durchgeführt. Die Lösung wird bei einer Temperatur von 35°C in den Wirbelschichtgranulator gesprüht. Zur Fluidisierung des Bettinhaltes wird Stickstoff in einer Menge von 110 kg/h eingeblasen. Die Eintrittstemperatur des Fluidisiergases beträgt 138°C. Die Temperatur des Abgases beträgt 80,5°C. Als Sichtgas wird ebenfalls Stickstoff in einer Menge von 11,5 kg/h mit einer Temperatur von 81°C zugeführt. Der Inhalt des Wirbelbettes beträgt ca. 450 g. Die Granulierleistung beträgt ca. 2 kg pro Stunde. Man erhält ein frei fließendes Granulat mit einem mittleren Teilchendurchmesser von 0,8 mm. Die Granulate sind rund und weisen eine sehr glatte Oberfläche auf.

### Beispiel 4 (Huhn):

In der in Beispiel "Erdbeere" beschriebenen Apparatur wird eine Granulierung einer Lösung bestehend aus 44 Gew.-% Wasser, 14 Gew.-% Gummi arabicum, 31 Gew.-% hydrolysierter Stärke (Maltodextrin DE 15-19) und 11 Gew.-% Huhn-Aroma durchgeführt. Die Lösung wird bei einer Temperatur von 30°C in den Wirbelschichtgranulator gesprüht. Zur Fluidisierung des Bettinhaltes wird Stickstoff in einer Menge von 130 kg/h eingeblasen. Die Eintrittstemperatur des Fluidisiergases beträgt 140°C. Die Temperatur des Abgases beträgt 91°C. Als Sichtgas wird ebenfalls Stickstoff in einer Menge von 16 kg/h mit einer Temperatur von 65°C zugeführt. Der Inhalt des Wirbelbettes beträgt ca. 650 g. Die Granulierleistung beträgt ca. 2 kg pro Stunde. Man erhält ein frei fließendes Granulat mit einem mittleren Teilchendurchmesser von 1,5 mm. Die Granulate sind rund und weisen eine mäßig glatte Oberfläche auf.

### Beispiel 5 (Himbeere):

In der in Beispiel "Erdbeere" beschriebenen Apparatur wird eine Granulierung einer Lösung bestehend aus 50 Gew.-% Wasser, 1 1 Gew.-% Gummi arabicum, 22,5 Gew.-% hydrolysierter Stärke (Maltodextrin DE 15-19) und 16,5 Gew.-% Himbeeraroma sowie einer geringen Menge Farbstoff durchgeführt. Die Lösung wird bei einer Temperatur von 32°C in den Wirbelschichtgranulator gesprüht. Zur Fluidisieung des Bettinhaltes wird Stickstoff in einer Menge von 110 kg/h eingeblasen. Die Eintrittstemperatur des Fluidisiergases beträgt 130°C. Die Temperatur des Abgases beträgt 84°C. Als Sichtgas wird ebenfalls Stickstoff in einer Menge von 9 kg/h mit einer Temperatur von 81°C zugeführt. Der Inhalt des Wirbelbettes beträgt ca. 300 g. Die Granulierleistung beträgt ca. 1,5kg pro Stunde. Man erhält ein frei fließendes Granulat mit einem mittleren Teilchendurchmesser von 0,5 mm. Die Granulate sind rund und weisen eine mäßig glatte Oberfläche (teilweise mit Sekundäragglomeraten) auf.

In derselben Apparatur wurden die zuvor erzeugten Granulate mit Boysenberryaroma (Lösung bestehend aus 50 Gew.-% Wasser, 11 Gew.-% Gummi arabicum, 22,5 Gew.-% hydrolysierter Stärke (Maltodextrin DE 15-19) und 16,5 Gew.-% Boysenaroma) gecoatet; als Bettvorlage werden 530 g vorgelegt. Durch Anheben der Sichtgasmenge auf 20 kg/h bei 90°C wird kein Material ausgetragen, d.h. das Coaten findet im Batchbetrieb statt. Die Lösung wird bei einer Temperatur von 26°C in den Wirbelschichtgranulator gesprüht. Die Temperatur des Zerstäubungsgases beträgt 30°C. Zur Fluidisierung des Bettinhaltes wird Stickstoff in einer Menge von 110 kg/h eingeblasen. Die Eintriftstemperatur des Fluidisiergases beträgt 130°C. Die Temperatur des Abgases beträgt 82°C. Man erhält ein frei fließendes Granulat. Die Feststoffpartikel sind rund. REM-Aufnahmen der Bruchflächen zeigen eine sehr gleichmäßige Beschichtung der Granulate.

### Beispiel 6 (Ethylbutyrat):

In der in Beispiel "Erdbeere" beschriebenen Apparatur wird eine Granulierung einer Lösung bestehend aus 38 Gew.-% Wasser, 15 Gew.-% Gummi arabicum, 34 Gew.% hydrolysierter Stärke (Maltodextrin DE 15-19) und 13 Gew.-% Ethylbutyrat durchgeführt. Die Lösung wird bei einer Temperatur von 38°C in den Wirbelschichtgranulator gesprüht. Zur Fluidisierung des Bettinhaltes wird Stickstoff in einer Menge von 125 kg/h eingeblasen. Die Eintrittstemperatur des Fluidisiergases beträgt 105°C. Die Temperatur des Abgases beträgt 66°C. Als Sichtgas wird ebenfalls Stickstoff in einer Menge von 10 kg/h mit einer Temperatur von 30°C zugeführt. Der Inhalt des Wirbelbettes beträgt ca. 1650 g. Die Granulierleistung beträgt ca. 1,4 kg pro Stunde. Man erhält ein frei fließendes Granulat mit einem mittleren Teilchendurchmesser von 0,5 mm bei einer Schüttdichte von 465 g/l. Die Granulate sind rund und weisen eine mäßig glatte Oberfläche auf.

### Beispiel 7 (Modellaromagemisch):

In der in Beispiel "Erdbeere" beschriebenen Apparatur wird eine Granulierung einer Lösung bestehend aus 50 Gew.-% Wasser, 4 Gew.-% Gummi arabicum, 36 Gew.-% hydrolysierter Stärke (Maltodextrin DE 15-19) und 10,0 Gew.-% Modellaromagemisch (Limonen:Ethylbutyrat:Phenylethanol = 1:1:1) durchgeführt. Die Lösung wird bei einer Temperatur von 22°C in den Wirbelschichtgranulator gesprüht. Zur Fluidisierung des Bettinhaltes wird Stickstoff in einer Menge von 125 kg/h eingeblasen. Die Eintrittstemperatur des Fluidisiergases beträgt 105°C. Die Temperatur des Abgases beträgt 59°C. Als Sichtgas wird ebenfalls Stickstoff in einer Menge von 14 kg/h mit einer Temperatur von 30°C zugeführt. Der Inhalt des Wirbelbettes beträgt ca. 700 g. Die Granulierleistung beträgt ca. 1,25 kg pro Stunde. Man erhält ein frei fließendes Granulat mit einem mittleren Teilchendurchmesser von 0,7 mm. Die Granulate sind rund und weisen eine rauhe Oberfläche auf.

In derselben Apparatur wurden die zuvor erzeugten Granulate mit Methylcellulose (wäßrige Lösung mit 2,0 Gew.-% Feststoff) Methocel A 15 LV (Dow Chemical) gecoatet; als Bettvorlage werden 480 g vorgelegt. Durch Anheben der Sichtgasmenge auf 20 kg/h bei 30°C wird kein Material ausgetragen, d.h. das Coaten findet im Batchbetrieb statt. Die Lösung wird bei einer Temperatur von 22°C in den Wirbelschichtgranulator gesprüht. Die Temperatur des Zerstäubungsgases beträgt 30°C. Zur Fluidisierung des Bettinhaltes wird Stickstoff in einer Menge von 120 kg/h eingeblasen. Die Eintrittstemperatur des Fluidisiergases beträgt 140°C. Die Temperatur des Abgases beträgt 81°C. Man erhält ein frei fließendes Granulat. Die Feststoffpartikel sind rund. REM-Aufnahmen der Bruchflächen zeigen eine sehr gleichmäßige und dünne Beschichtung der Granulate (5 Gew.-% Methylcellulose bezogen auf das Granulatgewicht).

### Einsatz der Granulate in der Anwendung

Die in den Beispielen erwähnten Aromen- und Riechstoffgranulate werden in den zu aromatisierenden Lebensmitteln (z.B. Instant-Getränkepulter, Aufgußteebeutel, Hartund Weichkaramellen, Weingummis, Backwaren, Nährmittel, Komprimate, Kaugummi Eiscreme, Eiscremecoating, gefüllte Schokoladenprodukte, Instant-Suppen und ―Saucen, gefrorene Fertiggerichte, wärmebehandelte Getränke, Suppen und Saucen, Mundhygieneprodukte wie Gebißreinigungstabletten und Zahnpasten) eingesetzt oder in den zu parfümierenden Kosmetik-, Hygiene-, Pharma, Seifen-, Detergentien- oder Haushaltsprodukten verwendet.

### Anwendungsbeispiele

### Beispiel 8 (Hart- und Weichkaramellen)

Ein blau gefärbtes Aromagranulat mit eingekapseltem Mint-Aroma wird zu 1 % in die heiße (140°C) Hartkaramellmasse bestehend aus Saccharose, Glucosesirup und Wasser eingemischt. Anschließend wird die noch heiße Masse in Formen gegossen.

Für die Herstellung von Weichkaramellen wird das Aromagranulat entsprechend in eine Masse, die Saccharose, Wasser, Glucosesirup, Fett, Fondant. Gelatine. Zitronensäure und einen Emulgator enthält, bei 120°C eingearbeitet. Anschließend wird die Masse auf einem Kühltisch auf unter 40°C abgekühlt und dann per Hand durch Ziehen belüftet.

### Vorteile:

- Durch die auffälligen Partikel kann ein optischer Effekt erzielt werden, der während der Verarbeitung und Lagerung erhalten bleibt.
- Es treten geringe Aromaverluste beim Verarbeitungsprozeß auf.
- Das Aroma ist an wenigen Stellen lokalisiert in der Matrix vorhanden und migriert nicht. Dadurch wird ein besonderer sensorischer Effekt erreicht (Hot Spots). In die aramellmatrix selbst kann ein anderes flüssiges Aroma gegeben werden womit ein sensorischer Doppeleffekt erzielt werden kann
- Das Ablutschverhalten der Hartkaramellen bleibt unverändert, die Partikel werde nicht als störend empfunden.

### Beispiel 9 (Geleefrüchte)

Ein rot gefärbtes Aromagranulat mit eingekapseltem Erdbeeraroma, das anschließend zusätzlich mit einem Fettüberzug versehen wurde, wird in die Masse für Geleefrüchte bestehend aus Wasser, Saccharose, Glucosesirup und Agar-Agar bei 70°C eingearbeitet. Anschließend wird die Masse in Formpuder gegossen.

### Vorteile :

- Durch die auffälligen Partikel kann ein optischer Effekt erzielt werden, der während der Verarbeitung und Lagerung trotz relativ hohem Wassergehalt in der Matrix erhalten bleibt.
- Es treten geringe Aromaverluste beim Verarbeitungsprozeß auf.
- Das Aroma ist an wenigen Stellen lokalisiert in der Matrix vorhanden und migriert nicht. Dadurch wird ein besonderer sensorischer Effekt erreicht (Hot Spots). In die Matrix selbst kann ein anderes flüssiges Aroma gegeben werden womit ein sensorischer Doppeleffekt erzielt werden kann.

### Beispiel 10 (Hartkekse)

Ein orange gefärbtes Aromagranulat mit eingekapseltem Käsearoma, das anschließend zusätzlich mit einem Fettcoating versehen wurde, wird in den Teig für Hartkekse eingearbeitet.

### Vorteile

- Es treten geringe Aromaverluste beim Backprozeß auf.
- Das Aroma ist an wenigen Stellen lokalisiert in der Matrix vorhanden und migriert nicht, dadurch wird ein besonderer sensorischer Effekt erreicht (Hot Spots). In die Matrix selbst kann ein anderes flüssiges Aroma gegeben werden womit ein sensorischer Doppeleffekt erzielt werden kann.

### Beispiel 11 (Kaugummi)

Ein Aromagranulat mit eingekapseitem Mintaroma wird in Kaugummimasse eingearbeitet.

### Vorteile:

- Hoher Aromaimpakt durch partielle Lokalisierung von hohen Aromakonzentrationen im Produkt. Die Freisetzung des Aromas erfolgt mechanisch beim Kauen

### Beispiel 12 (Eiscreme)

Ein orange gefärbtes Aromagranulat mit eingekapseltem Aprikosenaroma, das zusätzlich eine Umhüllung aus Fett enthält, wird in Eiscreme eingearbeitet.

### Vorteile:

- Durch die auffälligen Partikel kann ein optischer Effekt erzielt werden, der während der Lagerung der Eiscreme auch bei Temperaturschwankungen erhalten bleibt.
- Ein zusätzlicher Knuspereffekt kann selbst in Eiscreme als Lebensmittel mit relativ hoher Wasseraktivität realisiert werden.

### Beispiel 13 (Komprimate)

Ein Aromagranulat, das eingekapseltes Heidelbeeraroma überzogen mit einer 5%igen Schicht aus Methylcellulose enthält, wird zu 2 % in eine Pulvermischung aus Sorbit, Zitronensäure und Aspartam gegeben und über eine Tablettiermaschine zu Komprimaten verpreßt.

### Vorteile:

- Die Hygroskopizität der Pulvermischung wird deutlich herabgesetzt. Ein Anbacken an die Stempeloberflächen während des Verpressens tritt nicht mehr ein.

### Beispiel 14 (Vergleich der Verfahren)

Die Figur stellt einen Vergleich der unterschiedlichen Retentionsraten (Ordinate: Retention in %) einzelner Aromakomponenten aus einem Erdbeer-Aroma je nach der verwendeten Aromentechnologie dar. Die vertikalen Balken repräsentieren jeweils die einzelnen Aromakomponenten geordnet von links nach rechts nach abfallender Flüchtigkeit.

Deutlich ist zu erkennen, dass insbesondere für die leichtflüchtigen Komponenten (jeweils die Balken ganz links einer jeden Gruppierung) die Retention bei der erfindungsgemäßen kontinuierlichen Wirbelschichtgranulation 5 sehr gut ist. Damit wird erreicht, dass die Verhältnisse der Aromakomponenten untereinander nahezu unverändert bleiben. Das Geschmacksprofil entspricht dadurch weitgehend dem des flüssigen unverkapselten Aromas.

Die anderen dargestellten Technologien (1 = Adsorption; 2 = Sprühtrocknung; 3 = Agglomeration; 4 = Kompaktierung) fallen gegenüber der kontinuierlichen Wirbelschichtsprühgranulation in puncto Aromaprofilerhalt deutlich ab. Auch die Retention für das Aroma insgesamt ist bei der kontinuierlichen Wirbelschichtsprühgranulation am höchsten.

## Patentansprüche

1. Eingekapselte Aroma- und/oder Riechstoffzubereitungen, die mittels einer Wirbelschicht-Sprühgranulation erhältlich sind, bei der eine Aromen- und/oder Riechstoffzubereitung in ein Wirbelbett mit im Wirbelbett erzeugten Granulationskeimen eingesprüht wird und wobei die durchschnittliche Verweilzeit der eingesprühten Aromen- und/oder Riechstoffzubereitungen im Wirbelbett weniger als 20 Minuten beträgt.

2. Verfahren zur Herstellung von eingekapselten Aroma- und/oder Riechstoffzubereitungen mittels Wirbelschicht-Sprühgranulation, bei der eine Aromen- und/oder Riechstoffzubereitung in ein Wirbelbett mit darin erzeugten Granulationskeimen eingesprüht wird, **dadurch gekennzeichnet, dass** die durchschnittliche Verweilzeit der eingesprühten Aromen und/oder Riechstoffzubereitungen im Wirbelbett weniger als 20 Minuten beträgt.

3. Verfahren nach Anspruch 2, **dadurch gekennzeichnet, dass** das Verfahren kontinuierlich ausgeführt wird.

4. Verfahren nach einem der Ansprüche 2 bis 3, **dadurch gekennzeichnet, dass** das Wirbelbett eine Betthöhe von weniger als 10 cm hat.

5. Verfahren nach einem der Ansprüche 2 bis 4, **dadurch gekennzeichnet, dass** Granulat der gewünschten Korngröße mit einem Sichter aus dem Wirbelbett abgeschieden wird.

6. Verfahren nach einem der Ansprüche 2 bis 5, **dadurch gekennzeichnet, dass** das Granulat nach der Erzeugung durch Aufsprühen eines flüssigen Umhüllungsmaterials mit einer äußeren Schicht versehen wird.

7. Verfahren nach einem der Ansprüche 2 bis 6, **dadurch gekennzeichnet, dass** die Aromen- und/oder Riechstoffe in Form einer Emulsion, hergestellt durch Mischung der Aromen und/oder Riechstoffe mit Wasser und einem polymeren Trägerstoff, eingesetzt werden.

8. Verfahren nach einem der Ansprüche 2 bis 7, **dadurch gekennzeichnet, dass** als polymere Trägerstoffe hydrolisierte oder modifizierte Stärken und als Hydrokolloide Gummi Arabikum oder Mischungen hiervon eingesetzt werden.

9. Verfahren nach einem der Ansprüche 7 bis 8, **dadurch gekennzeichnet, dass** die Aromen- und/oder Riechstoffemulsion als Zusatzstoffe, Lebensmittel- oder Kosmetik-Farbstoffe, Süßstoffe, Antioxidantien, Genusssäuren, geschmacksbeeinflussende Stoffe, Vitamine, Mineralstoffe und/oder Saftkonzentrate enthält.

10. Verfahren nach einem der Ansprüche 7 bis 9, **dadurch gekennzeichnet, dass** die Aromen- und/oder Riechstoffemulsion auch Zusatzstoffe und Zutaten wie Lebensmittel- oder Kosmetik-Farbstoffe, Süßstoffe, Zuckeraustauschstoffe, Antioxidantien, Genusssäuren, geschmacksbeeinflussende Stoffe, Vitamine, Mineralstoffe und/oder Saftkonzentrate enthält.

## Claims

1. Encapsulated flavour and/or fragrance preparations that are obtainable by means of fluidised bed spray granulation, in which a flavour and/or fragrance preparation is sprayed into a fluidised bed containing granulation nuclei produced in the fluidised bed and where the average residence time in the fluidised bed of the flavour and/or fragrance preparations sprayed in is less than 20 minutes.

2. Method for the production of encapsulated flavour and/or fragrance preparations by means of fluidised bed spray granulation, in which a flavour and/or fragrance preparation is sprayed into a fluidised bed containing granulation nuclei produced therein, **characterised in that** the average residence time in the fluidised bed of the flavours (sic) and/or fragrance preparations sprayed in is less than 20 minutes.

3. Method according to Claim 2, **characterised in that** the method is carried out continuously.

4. Method according to one of Claims 2 to 3, **characterised in that** the fluidised bed has a bed height of less than 10 cm.

5. Method according to one of Claims 2 to 4, **characterised in that** granules of the desired particle size are separated off from the fluidised bed using a sifter.

6. Method according to one of Claims 2 to 5, **characterised in that**, after production, the granules are provided with an outer coating by spraying on a liquid coating material.

7. Method according to one of Claims 2 to 6, **characterised in that** the flavours and/or fragrances are used in the form of an emulsion prepared by mixing the flavours and/or fragrances with water and a polymer carrier.

8. Method according to one of Claims 2 to 7, **characterised in that** the polymer carriers used are hydrolysed or modified starches and the hydrocolloids used are gum arabic or mixtures thereof.

9. Method according to one of Claims 7 to 8, **characterised in that** the flavour and/or fragrance emulsion contains food dyes or cosmetics dyes, sweeteners, antioxidants, edible acids, substances that influence the taste, vitamins, minerals and/or juice concentrates as additives.

10. Method according to one of Claims 7 to 9, **characterised in that** the flavour and/or fragrance emulsion also contains additives and ingredients such as food dyes or cosmetics dyes, sweeteners, sugar substitutes, antioxidants, edible acids, substances that influence the taste, vitamins, minerals and/or juice concentrates.

## Revendications

1. Préparations encapsulées d'arômes et/ou de parfums, qui peuvent être obtenues par granulation par pulvérisation en lit fluidisé dans laquelle on pulvérise une préparation d'arômes et/ou de parfums dans un lit fluidisé avec des germes de granulation produits dans le lit fluidisé, le temps de séjour moyen des préparations d'arômes et/ou de parfums pulvérisées dans le lit fluidisé étant inférieur à 20 minutes.

2. Procédé de production de préparations encapsulées d'arômes et/ou de parfums par granulation par pulvérisation en lit fluidisé, dans lequel on pulvérise une préparation d'arômes et/ou de parfums dans un lit fluidisé avec des germes de granulation produits dans le lit fluidisé, **caractérisé en ce que** le temps de séjour moyen des préparations d'arômes et/ou de parfums pulvérisées dans le lit fluidisé est inférieur à 20 minutes.

3. Procédé selon la revendication 2, **caractérisé en ce que** le procédé s'effectue en continu.

4. Procédé selon l'une des revendications 2 à 3, **caractérisé en ce que** le lit fluidisé a une hauteur de lit inférieure à 10 cm.

5. Procédé selon l'une des revendications 2 à 4, **caractérisé en ce que** l'on sépare des granulés ayant la grosseur de grains désirée à l'aide d'un classificateur à la sortie du lit fluidisé.

6. Procédé selon l'une des revendications 2 à 5, **caractérisé en ce que**, après la production, les granulés sont munis d'une couche externe par pulvérisation d'un matériau d'enrobage liquide.

7. Procédé selon l'une des revendications 2 à 6, **caractérisé en ce que** l'on utilise les arômes et/ou les parfums sous forme d'une émulsion préparée par mélange des arômes et/ou des parfums avec de l'eau et un support polymère.

8. Procédé selon l'une des revendications 2 à 7, **caractérisé en ce que** l'on utilise comme supports solides des amidons hydrolysés ou modifiés et comme hydrocolloïdes de la gomme arabique ou leurs mélanges.

9. Procédé selon l'une des revendications 7 à 8, **caractérisé en ce que** l'émulsion d'arômes et/ou de parfums contient comme additifs des colorants alimentaires ou cosmétiques, des édulcorants, des antioxydants, des acides alimentaires, des substances influençant le goût, des vitamines, des substances minérales et/ou des concentrés de jus.

10. Procédé selon l'une des revendications 7 à 9, **caractérisé en ce que** l'émulsion d'arômes et/ou de parfums contient aussi des additifs et des ingrédients comme des colorants alimentaires ou cosmétiques, des édulcorants, des substituts du sucre, des antioxydants, des acides alimentaires, des substances influençant le goût, des vitamines, des substances minérales et/ou des concentrés de jus.
